# EUROPEAN PATENT APPLICATION

(11) **EP 4 483 779 A1**
(43) Date of publication of application: **01.01.2025**
(21) Application number: 23182137.2
(22) Date of filing: 28.06.2023
(51) Int. Cl.: A61B 1/005

(54) **AN ENDOSCOPE WITH WIRE PIPES FOR GUIDING STEERING WIRES**

(71) Applicant: Ambu A/S, 2750 Ballerup (DK)
(72) Inventor: PALACIOS, Irene Rivas, 2100 Copenhagen Ø (DK); HJORTLUND, Jonas, 2300 Copenhagen S (DK); HANSEN, Michael Kappler, 2665 Vallensbæk (DK); CHRISTENSEN, Martin Johst, 2100 Copehagen Ø (DK)
(74) Representative: AWA Denmark A/S

(57) **Abstract**

An endoscope (1) comprising steering wires (25) for bending a bending section (20), a steering wire actuator (32), and a wire pipe base (70) comprising a first and a second channel (18). The endoscope comprising a first and a second wire pipes (26) fixated in the first and the second channel (18) and crossing proximally of the wire pipe base (70) at a point (C) intermediate the wire pipe base (70) and the steering wire actuator (32).

## Description

### Technical Field

The present disclosure relates to an endoscope comprising an insertion cord including a bending section. More specifically, bending of the bending section is controlled by steering wires running inside wire pipes.

### Background

Flexible endoscopes for medical purposes are often provided with a bending section at a distal end of an insertion tube. This enables the user of the endoscope to maneuver the distal tip of the endoscope inside the human anatomy, such as in the airways, in the kidneys or the gastro-intestinal system, and e.g., to study or perform procedures at tissue of interest. The bending section is typically bent by pulling steering wires. If the endoscope is a 2-way bending endoscope, i.e., bending in two opposite directions but in the same plane, the bending section will typically be controlled by two steering wires, which are controlled by one steering wire actuator arranged in the handle of the endoscope, allowing the user to bend the bending section by adjusting a bending lever. If the endoscope is a four-way bending endoscope, i.e., also bending in two opposite directions in a second plane perpendicular to the first mentioned plane, the bending section will typically be controlled by four steering wires, where one steering wire actuator controls two steering wires for bending in the first plane, and the other steering wire actuator controls the two other steering wires for bending in the second plane. Typically, each steering wire actuator is controlled by a rotational wheel on the endoscope handle.

The steering wires will pass from the handle to the proximal end of the bending section inside wire pipes, which are tubes having limited compressibility. Thereby so-called Bowden cables are formed. The wire pipes are at their proximal end connected inside the handle. At the distal end of the wire pipes, they are connected to the proximal end of the bending section, whereas the steering wires continue through the bending section.

For single-use endoscopes the wire pipe base, the steering wire actuator, and the fastening of the steering wires to the steering wire actuator needs to facilitate simple manufacturing and assembly and needs to facilitate adjustment of the tension on the steering wires achieving optimal maneuverability of the bending section. Also, for single-use endoscopes it may be preferred to apply the same component in different types of endoscopes, when possible, as this reduces costs for manufacturing by reducing the overall number of different parts. Therefore, it may be advantageous use an attachment mechanism suitable for different sizes.

### Summary

It is an object of the present disclosure to provide an endoscope comprising a steering wire actuator, a wire pipe base comprising a first and a second channel, a first and a second wire pipes fixated in the first and the second channel and crossing proximally of the wire pipe base at a point C intermediate the wire pipe base and the steering wire actuator.

The endoscope may comprise an insertion cord including an insertion tube and a bending section; a handle comprising the steering wire actuator and the wire pipe base; a first steering wire and a second steering wire both connected to the steering wire actuator and running through the insertion cord so that manipulation of the steering wire actuator causes bending of the bending section. The first and the second wire pipe extend from the wire pipe base to a distal end of the insertion tube, the first and the second steering wire running inside the first and the second wire pipe, respectively.

The advantage of the wire pipe base is that the steering wires between the wire pipe base and the steering wire actuator can be arranged to propagate away from inner surfaces of the handle shells. This leaves more space for e.g., tools fixating steering wire ends to the steering wire, e.g., by crimping, during the manufacturing of the endoscope.

Further, this design of the wire pipe base ensures that the same wire pipe base can be applied in different types of endoscopes having different dimensions of the steering wire actuator.

In the following, the expression "distal" is defined to be in the direction toward the patient, and "proximal" is defined to be in the direction away from the patient. For the handle of the endoscope, the distal end will be the end where the insertion tube is connected, and the proximal end is the opposite end. Further, the expression "handle" may be a positioning interface, or interface, which functions to control the position of the insertion cord. The handle, or positioning interface, may be an interface operated by a robotic arm, or it may be a handle operated by the hand of an endoscope user.

For this disclosure one steering wire is counted as one passage from the steering wire actuator (or roller) to the distal end of the bending section. I.e., if the same unbroken steering wire continues from the steering wire actuator to the distal end of the bending section and back to the steering wire actuator, and one part is applied for bending for example to one side and the other part is applied for bending to the opposite side, this is counted as two steering wires, e.g., first and second steering wires.

In a variation of the present embodiment, the first and the second channel each have a centerline extending in parallel with the first and second axis, respectively. This has the advantage of simplifying the positioning of the first and second wire pipes in a selected mutual angle, during manufacturing of the endoscope.

In a variation of the present embodiment, the handle comprises an encasement having a wall part to which the wire pipe base is attached. Such an encasement is often present in an endoscope handle to seal and protect an electronic circuit board in relation to liquids. Combining the two parts has the advantage of reducing the number of different parts which needs to be handled and assembled during manufacturing. Also, as the encasement is typically larger than the wire pipe base, fixation of the wire pipe base to the encasement enables a strong and stable connection between the wire pipe base and the handle shells. Such a strong and stable connection is important for the bending performance when manipulating a bending lever. The wall part of the encasement may have a planar surface, on the part exterior to the encasement, to twhich the wire pipe base is attached.

In a variation of the present embodiment, the wall part of the encasement and the wire pipe base are made in one piece of material. This may be done by molding the two parts in one piece of material, whereby the wall part and the wire pipe fastener are fused together. This will reduce the costs by combining two molding processes into one. Further, combining the two parts may save space inside the handle, as separate means, such as glue or screws, for fixation of the parts to the handle shells, can be avoided.

In a variation of the present embodiment, a slot is placed at the position C where the first and the second steering wires are crossing each other. This slot has the advantage of forcing the steering wires to cross at the position C, also when another size of the roller is applied for a different endoscope model. Having the crossing at the same position between different endoscope models, may streamline the manufacturing process.

In a further variation of the present embodiment, a structure forming the slot projects from and is attached to the outer wall part of the encasement. When these two parts are attached to each other, the assembly process of the endoscope handle or actuator may be faster. The structure forming the slot may comprise two walls spaced apart by the slot.

In a variation of the present embodiment, the outer wall part of the encasement, the wire pipe base, and the structure forming the slot, are made in one piece of material, e.g., by fusing the parts together, e.g., in a molding process forming the parts in one single piece of material. This will reduce the number of parts, thereby making the manufacturing cheaper.

In a variation of the present embodiment, the channels are arranged in parallel displaced planes. Preferably, the bottoms of the channels are arranged in parallel but displaced planes. Both planes may be perpendicular to, or substantially perpendicular to, an axis of rotation of a roller being part of the steering wire actuator. One advantage of the displaced planes is that it ensures that the two steering wires will not get into contact between the wire pipe base and the steering wire actuator.

In a variation of the present embodiment, the wire pipes are extending proximally from the wire pipe base and passed the position C. An advantage of this is that if the steering wires should get close to each other in the position C, there is no possibility that they will slide against one another, and thereby reduce the bending performance of the endoscope.

In a variation of the present embodiment, the distance between the steering wire actuator and the position C is larger than the distance between the wire pipe base and the position C, preferably the distance between the steering wire actuator and the position C is at least the double of the distance between the wire pipe base and the position C. This is an advantage as the fixation of a steering wire end to the steering wire itself preferably takes place between the steering wire actuator and the position C. So, this will leave more space for any tool, E.g., crimping tool, to be applied for this process.

In a variation of the present embodiment, the channels each having at least one inner surface provided with ribs, the ribs are preferably extending in a direction being transverse to the direction of the first or second axis of the wire pipe placed in the respective channel. The ribs will improve the attachment of a glue inside the channel.

In a variation of the present embodiment, the wire pipe base is provided with a lid part covering at least part of the first channel and of the second channel. The lid part may be provided with holes for application of glue into the channels after placement of the lid part. The lid part may be made from a transparent polymer, facilitating curing of applied glue by UV light. The lid part may be applied for pressing the wire pipes into the correct position or for keeping the wire pipes in the correct position during the gluing process.

In a second aspect the disclosure relates to a method for assembling an endoscope, the method comprising the steps of:
- providing a handle shell part,
- arranging a wire pipe base and an actuator in the handle shell part,
- connecting a first steering wire and a second steering wire to the roller,
- connecting wire pipes, through which the steering wires are inserted, to the wire pipe base,
- adjusting the tension for each steering wire and following fixating the proximal end of the first steering wire to the first steering wire and fixating the proximal end of the second steering wire to the second steering wire.

In a third aspect the disclosure relates to a system comprising an endoscope according to the first aspect and variations thereof, a monitor and a control unit.

### Brief description of the figures

The above-mentioned embodiments and variations, features and advantages thereof will be further elucidated by the following illustrative and nonlimiting detailed description of embodiments disclosed herein with reference to the appended drawings, wherein:
Fig. 1 shows an endoscope and a monitor with a control unit.
Fig. 2 shows an example of a steering wire actuator, here in the form of a roller, and a wire pipe base.
Fig. 3 shows a distal end of an endoscope including a bending section.
Fig. 4 shows a handle shell part of an endoscope with a wire pipe base.
Fig. 5 shows an enlarged view of the variation of a wire pipe base from fig. 4.
Fig. 6 shows the variation from fig. 5.
Fig. 7 shows the variation from fig. 6 from a different view
Fig. 8 shows the variation from fig. 5-7 illustrating the angle between the first and second axis along which the wire pipes extend.
Fig. 9 shows a wire pipe base provided with a lid part.
Fig. 10 shows the wire pipe base of fig. 9 with the lid part removed.
Fig. 11 shows the lid part from the opposite site than in fig. 9.
Fig. 12 shows the encasement for electronics with wire pipe base attached to a major wall part.

In the drawings, corresponding reference characters indicate corresponding parts, functions, and features throughout the several views. The drawings are not necessarily to scale and certain features may be exaggerated in order to better illustrate and explain the disclosed embodiments. For simplicity, this disclosure will focus on a two-way bending endoscope, but the disclosure is relevant for, and covers, also a four-way bending endoscope.

### Detailed description

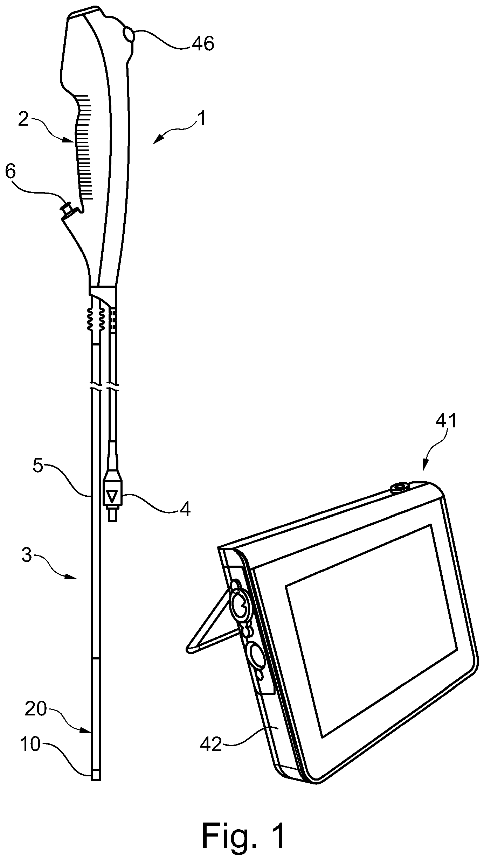

Fig. 1 illustrates an endoscope 1, which comprises a handle 2, an insertion cord 3 and an electrical cable with a connector 4 for connecting the endoscope 1 to a monitor 41. The insertion cord 3 is the part to be inserted into a body lumen during an endoscopic procedure. The insertion cord comprises a distal tip 10, a bending section 20 and a main tube 5. The handle 2 may comprise an entrance to a working channel 6 running through the insertion cord to the distal tip. The handle also comprises a bending lever 46, which can be used for bending the bending section.

The distal tip 10 comprises a camera and light emitters, e.g., in the form of one or more LEDs or the end of an optical light fiber.

The monitor 41 may be combined with an electronic circuit for receiving and processing the image stream from the camera as well as a processor for image processing, user interface, storage of images etc. But the monitor part and the electronic circuit and processor part may also be separate parts. The electronic circuit and the processor part are also referred to as a control unit 42.

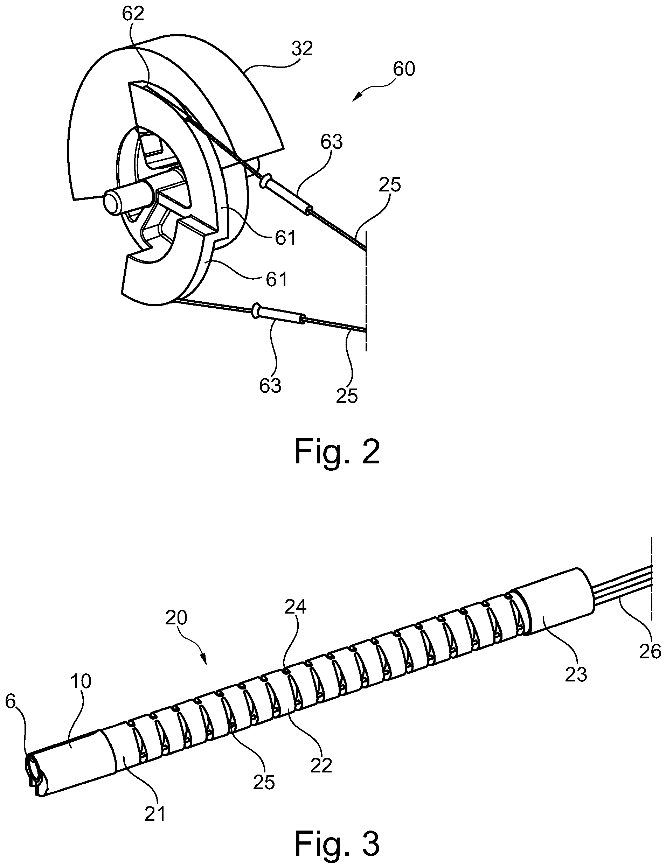

Fig. 2 shows a schematic example of a roller 32 for a steering wire actuator 60 configured for bending the bending section by pulling the steering wires 25. The steering wires 25 are moved by rotation of wire drum curved surfaces 61, which are a circular arc surfaces or a curved surface placed on the roller 32 and supporting the steering wires. In the example, one end of the steering wire has been drawn through a fixing structure 62 and bent back and fastened to itself by a crimp 63. The fixing structure may be a small hole (not shown) through which the steering wire is threaded. The system in fig. 2 works for a two-way bending endoscope. For a four-way bending endoscope two independently rotatable wire drums may be applied.

Fig. 3 shows a distal end of the insertion cord 3 of an endoscope, where the bending cover (not shown) typically covering the bending section 20 part and often makes this part watertight, has been removed. The bending section is attached to the main tube 5 in the proximal end, and to the distal tip 10 in the distal end. In this example the bending section is molded in one piece and comprises a number of segments including a proximal end segment 23, a distal end segment 21, and intermediate segments 22. The proximal end segment 23 is connected to the distal end of the main tube 5. The segments are held together by hinges 24, so that the segments can be bent relative to each other by manipulation of the steering wires 25. An example of such a bending section molded in one piece can be seen in US 10,321,804 B2, which is incorporated herein by reference in its entirety.

Alternatively, the bending section 20 could be extruded in a relatively soft and resilient material, e.g., a foam-like material, with lumens for steering wires, electrical wires and the tubes passing through.

Steering wires 25 are connected in a fixed connection to the distal end, e.g., the distal end bending segment 21. I.e., the steering wire is preferably not movable in relation to the distal end bending segment. Between the handle and the distal end of the main tube 5, or the proximal end of the bending segment 23, the steering wire 25 is guided in a wire pipe 26 which is secured in the handle 2 distal to the steering wire actuator 60. Other designs of the bending section are also possible.

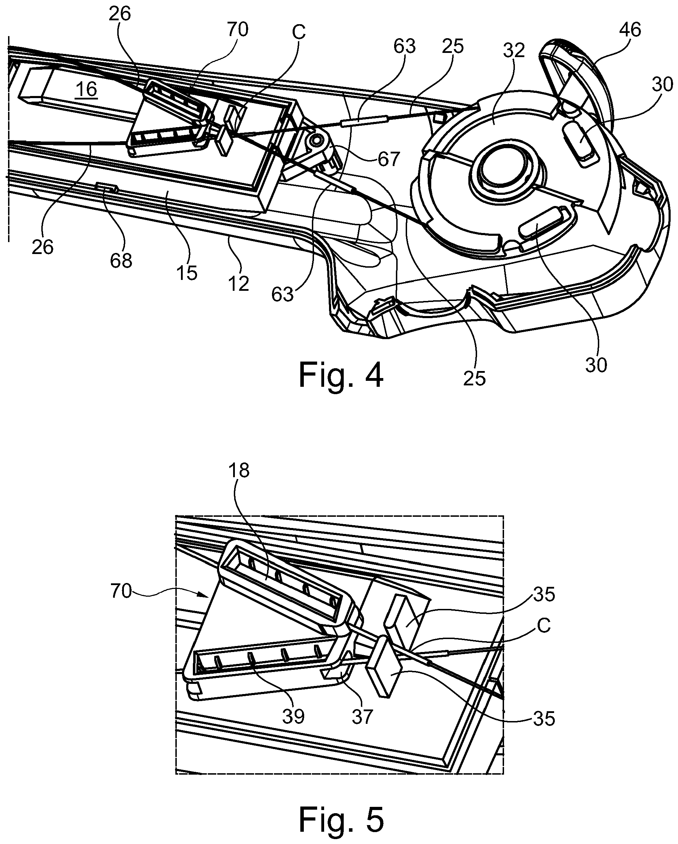

Fig. 4 shows an example of a part of an endoscope handle 2 with one handle shell removed. Illustrated in the one remaining handle shell 12 is a roller 32 with a bending lever 46 attached. Also, an encasement 15 for e.g., electronics is shown. The encasement 15 is connected to the handle shell 12 and may have the form of a box. The encasement 15 may be watertight e.g., to protect enclosed electronics. The encasement 15 may have a wall part to which the wire pipe base 70 is attached as shown in fig. 4.

The encasement 15 may be attached to the handle shell by application of parts for press fits 67 and/or snap fits 68. This facilitates a simple assembly process of the endoscope. The encasement may be assembled from two parts and may comprise apertures for e.g., electrical wire connections. The encasement may be a box that is a separate part and comprises a two major walls, e.g. a bottom wall and a lid, and circumferential wall(s). Alternatively, some walls of the encasement are part of the shell and a lit is press-fit to enclose an inner space where the electronic circuit board is arranged.

In an example the encasement 15 has one part comprising one major surface also comprising the wire pipe base 70 and circumferential wall. An opposite major surface is a part of a handle shell 12. In this way the encasement 15 may be formed from two molded parts, where one part is also a handle shell part, and the other part also comprises the wire pipe base 70.

It is further illustrated in fig. 4 how the steering wires 25 are connected to the roller 32. Each of the two steering wire ends are connected around the hooks 30 and each steering wire end is guided back and secured to itself by crimps 63. The two steering wires 25 are crossing in a position or point C placed between the wire pipe base 70 and the roller 32. In the example shown in fig. 4 and 5 the steering wires has entered the wire pipes proximal to the point C of crossing. The wire pipes could also be ending between the wire pipe base and the point C. The point C, or position of crossing, is the point between the wire pipe base and the roller where the distance between the two steering wires is minimum.

The wire pipes 26 are fixated in the wire pipe base 70, and the steering wires 25 will move in the wire pipes 26 when the roller 32 is rotated e.g., by manipulation of the bending lever 46.

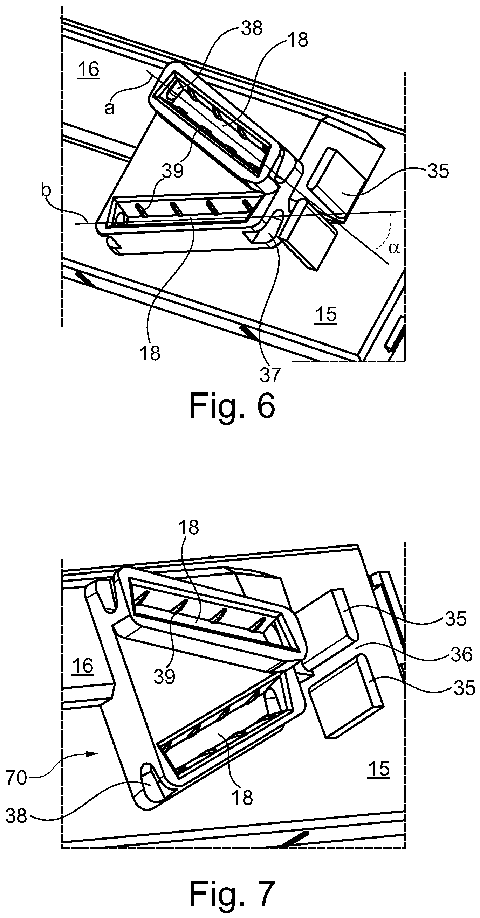

An example of the wire pipe base 70 is shown in fig. 5-8, where fig. 5 is an expanded view from fig. 4. The wire pipe base has two channels 18 or grooves or passages, adapted for the wire pipes 26 to pass through and being fixedly connected to the wire pipe base 70, which again is in a fixed connection to the handle of the endoscope. Each channel 18 has a proximal opening 37 and a distal opening 38. Each channel 18 may have one center axis a, b extending along the length of the channel. When the axes a, b are extended proximal the wire pipe base 70, the two axes a, b may cross each other between the wire pipe base 70 and the roller 32. The angle α between the two axes a, b will depend on the diameter of the roller 32 and the distance between the roller and the wire pipe base 70. The angle α may be in the range 20° - 50°, or in the range 30° - 40°.

The fixed connection of the wire pipes 26 inside the channels may be provided by gluing. The glue could preferably have a viscosity so that it will not flow out of the channels e.g., through the openings 37, 38, before it is cured. A glue which can be hardened by application of UV light may be used.

The wire pipe base is preferably made from a polymer material, such as polycarbonate (PC) or MABS. Both materials may be transparent, which is an advantage if a glue to be hardened by UV light is applied. Other polymers can also be applied, e.g. ABS if the transparency is not needed. If a polymer is applied to which it is difficult to achieve good adherence of the glue, the channel may have a geometry such that the hardened glue forms an anchor. Both the shape of the proximal openings 37, and the presence and the shape of ribs 39 may be used for providing such an anchor effect.

Press fit of the wire pipes 26 may also be applied for fixation of these to the wire pipe base 70. In that case the ribs 39 could be designed to exert a sufficient pressure on the wire pipes. This would imply that the channel 18 with the ribs should be designed for one specific outer diameter of the wire pipes. The advantage of the glued solution is that the same channel, with or without ribs; can be applied for different outer diameters of wire pipes. Fixation by a pressure fit has the advantage that the gluing process is avoided.

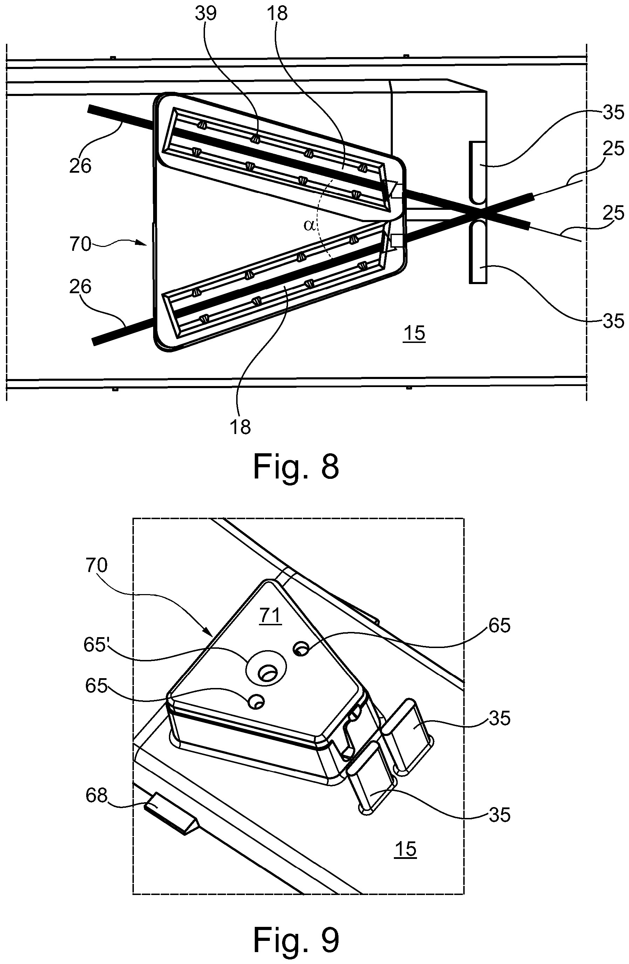

If a press fit is applied for fixation of the wire pipes 26 in the wire pipe base 70, ribs 39 could be added also to the bottom of the two channels 18 (this is not shown). Also, a cover could be placed on the top of the channels, e.g. as shown in fig. 9. This cover could be provided with ribs 39 extending into the channels 18 for improving the press fit of the wire pipes 26.

It is seen from fig. 5-7 that the bottoms of the two channels are not necessarily in the same plane or at the same level. In this example, if two planes are defined, one for each of the bottoms of the two channels, these planes could be parallel but may also be displaced relative to each other. The displacement could be in the range 1-3 mm, preferably in the range 1.5 - 2.5 mm. A similar displacement may be made between the two fixing structures 62 for attachment of the steering wires 25 on the roller 32, such as the two hooks 30 on the roller 32 to which the steering wires are connected in fig. 4. However, the displacement between the points of fixating the two steering wires at the roller may not necessarily have the same distance as the displacement of the bottoms of the two channels but may preferably be within a similar size range.

This displacement has the advantage that the two steering wires will not be touching each other. This is important if the proximal ends of the wire pipes are placed distal to the point C of crossing, such that the friction of the steering wires are not increased. The displacement can also be an advantage in the arrangement of all the parts in the handle. For example, the encasement 15 shown in fig. 4-8 are provided with an extending part 16 leaving more space inside the encasement. This more space may be used for the cable connecting the handle electronics to the monitor 41 and control unit 42.

Fig. 5 shows that two projections 35 in the form of walls, plates or rods, are placed distal to the wire pipe base 70 and forms a slot 36 between them close to the point C where the steering wires are crossing. The projections will preferably have a curved surface facing the slot. The distance between the proximal end of the wire pipe base and the distal side of the projections 35 may be between 2-8 mm, preferably between 3-6 mm.

The purpose with the slot 36 is to limit the position of the point C where the steering wires are crossing. Such a fixed or substantially fixed position of the point C to a specific distance from the proximal end of the wire pipe base in the direction of the roller 32, facilitates that the same design of the wire pipe base 70 may be applied with different diameters of the roller. This means that the same wire pipe base component may be used for different types of endoscopes, e.g. both bronchoscopes, cystoscopes and ureteroscopes, whereas the roller component often may need to be of different design as the requirements for bending performance between different procedures may be very different. Bending performance involves parameters such as maximum bending angle, length of bending section and the relationship between movement of bending lever and degrees of bending of the distal tip.

The slot 36 may be formed by two similar projections 35 or walls as shown in fig. 5-10. The slot may also be formed by two different walls, e.g., having different length of projection from the encasement 15. Also, the walls may differ in thickness and shape.

Reusing the wire pipe base part 70 between different types of endoscopes, and also having the same point C of crossing steering wires, facilitates a simpler manufacturing and enables that the same manufacturing equipment may be applied for the different types of endoscopes. Having the same point C of crossing makes it possible to use the same equipment for fixation of the steering wire end to the steering wire itself, by crimping. The crimping tool may be in one position. The handle shells with wire pipe base, roller, steering wires and wire pipes, are mounted in fixtures with the correct tension of the steering wires adjusted. The fixtures with handle shells are moved consecutively to the crimping tool where the crimping is done for both steering wires.

Fig. 9 shows a variation where the wire pipe base 70 is provided with a lid part 71 covering the channels 18. The lid part may be provided with glue holes 65 for application of glue for fixating the wire pipes 26 in the channels 18. Glue may also be applied through a glue hole 65' for securing the lid part 71 to the wire pipe base 70. The lid part 71 may be made from a transparent polymer to enable curing of the glue by UV light.

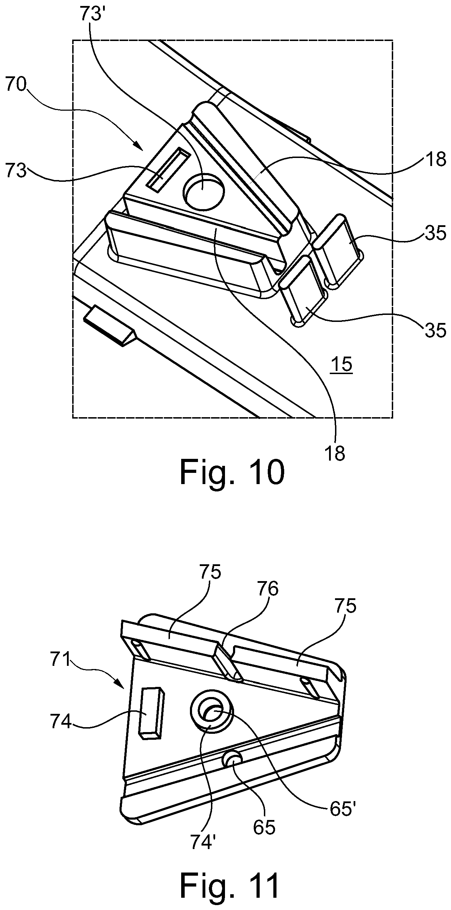

Fig. 10 shows the variation of fig. 9, but with the lid part 71 removed. Here, the channels 18 are without ribs, but ribs may be provided if considered expedient. It is seen that the wire pipe base 70 comprises indent areas 73, 73' for receiving corresponding alignment taps 74, 74' (see fig. 11) on the lid part 71.

Fig. 11 shows the lid part 71 which in fig. 9 is attached on the top of the wire pipe base 70 and closing the channels 18. Fig. 11 shows the side of the lid part 71 which is facing the wire pipe base 70 when attached to this. The lid part 71 also comprises two extending wall parts 75 for entering the channel 18 In which the wire pipe 26 is intended to be placed closest to the encasement 15, in order to position the wire pipe 26. The two wall parts 75 are spaced apart and thereby forms a passage 76 for glue between them. When the lid part 71 is arranged on the wire pipe base 70, the two wall parts 75 will extend into the channel 18 and glue can be applied into a glue hole and through the passage 76 for glue into contact with the wire pipe 26.

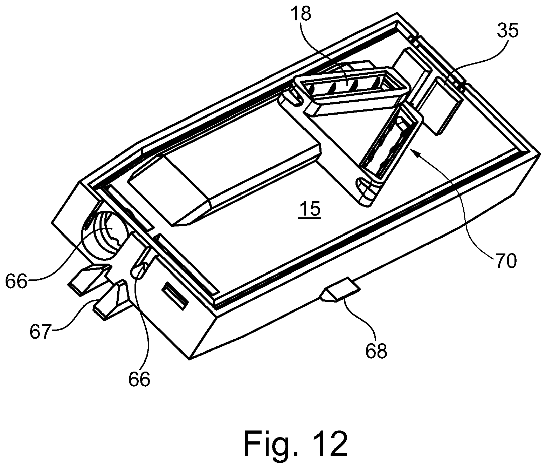

Fig. 12 shows the encasement 15 with the wire pipe base 70 arranged on a major wall part. This major wall part may be connected to the rest of the encasement by a glued sealing (not shown). Openings 66 for electrical cables are illustrated. Also, parts for press fit 67 and snap fit 68 are shown. Also, projections 35 forming the slot 36 are shown extending from the major wall part of the encasement 15.

In different examples of a wire pipe base 70 different measures may be applied. In one example the channels both have a width of approximately 2 mm, with around 1.5 mm at the bottom around 2.4 mm at the top of the channel. The length of each channel is in one example in the range 13 - 19 mm. The distance between the two wire pipes 26 at their exit at the proximal end of the wire pipe base 70 may be 2 - 5 mm. The distance between the two wire pipes 26 at the distal entrance to the wire pipe base may be 10 - 15 mm. The width of the slot opening 36 formed between the projections 35 may in an example be approximately 1-2 mm. In the example shown in fig. 4 the distance between the point where a wire pipe exits the wire pipe base proximal end and the point at which the corresponding steering wire steering wire contacts the wire drum surface of the roller, is around 55 - 65 mm. In an example the wire pipes are extending proximally from the wire pipe base and passed the position C by a distance of 2 - 20 mm, preferably 4-15 mm.

### List of references

| | |
|---|---|
| 1 endoscope | 38 distal opening |
| 2 handle | 39 rib |
| 3 insertion cord | 41 monitor |
| 4 electrical cable with plug | 42 control unit |
| 5 main tube | 46 bending lever |
| 6 working channel | 60 steering wire actuator |
| 10 distal tip | 61, 61' wire drum curved surface |
| 12 handle shell | 62 fixing structure |
| 15 encasement | 63 crimp |
| 16 extending part | 65, 65' glue hole |
| 18 channel | 66 cable opening |
| 20 bending section | 67 press fit part |
| 21 distal end segment | 68 snap fit part |
| 22 intermediate segment | 70 wire pipe base |
| 23 proximal end segment | 71 lid part |
| 24 hinges | 73, 73' indent areas |
| 25 steering wire | 74, 74' alignment taps |
| 26 wire pipe | 75 wall part |
| 30 hook | 76 passage for glue |
| 32 roller | α angle between channels |
| 35 projections | C point of crossing steering wires |
| 36 slot | |
| 37 proximal opening | |

## Claims

1. An endoscope comprising:
- an insertion cord including a main tube and a bending section distal to the main tube;
- a handle comprising a steering wire actuator and a wire pipe base, the wire pipe base including a first and a second channel;
- a first steering wire and a second steering wire both connected to the steering wire actuator and running through the insertion cord so that manipulation of the steering wire actuator causes bending of the bending section;
- a first and a second wire pipe extending from the wire pipe base to a distal end of the main tube, the first and the second steering wire running inside the first and the second wire pipe, respectively;
wherein the first and the second wire pipes are fixated in the first and the second channel of the wire pipe base, the first and the second wire pipe extending proximally of the wire pipe base along a first and a second axis, respectively, and
wherein the first and the second axis cross each other in a position (C) between the wire pipe base and the steering wire actuator.

2. The endoscope according to claim 1, wherein the first and the second channel each having a centerline extending in parallel with the first and second axis, respectively.

3. The endoscope according to any one of the previous claims, wherein the endoscope comprising an encasement located within the handle, the encasement comprising a wall part, the wire pipe base connected to and extending from the wall part.

4. The endoscope according to claim 3, wherein the wall part of the encasement and the wire pipe base are made in one piece.

5. The endoscope according to any one of the previous claims, further comprising a structure defining a slot, wherein the slot is placed at the position (C) where the first and the second steering wires cross.

6. The endoscope according to claim 5, wherein the structure forming the slot projects from and is attached to the wall part of the encasement.

7. The endoscope according to claim 6, wherein the wall part of the encasement, the wire pipe base, and the structure forming the slot, are made in one piece.

8. The endoscope according to claim 5, wherein the structure forming the slot comprises two walls spaced apart by the slot.

9. The endoscope according to any one of the previous claims, wherein the channels are arranged in parallel displaced planes.

10. The endoscope according to any one of the previous claims, wherein the wire pipes extend proximally from the wire pipe base and passed the position (C), or through the slot.

11. The endoscope according to any one of the previous claims, wherein a distance between the steering wire actuator and the position (C) is larger than a distance between the wire pipe base and the position (C), preferably the distance between the steering wire actuator and the position (C) is at least the double of the distance between the wire pipe base and the position (C).

12. The endoscope according to any one of the previous claims, wherein the channels are defined by walls, where ribs extend from at least one of these walls, the ribs are preferably extending in a direction transverse to the direction of the first or second axis of the wire pipe placed in the respective channel.

13. The endoscope according to any one of the previous claims, wherein the wire pipe base is provided with a lid part covering at least part of the first channel and of the second channel.

14. A method for assembling an endoscope according to any one of the previous claims, comprising the steps of:
- providing a handle shell part,
- arranging a wire pipe base and a roller in the handle shell part,
- connecting a first steering wire and a second steering wire to the roller,
- connecting wire pipes, through which the steering wires are inserted, to the wire pipe base,
- adjusting the tension for each steering wire and following fixating the proximal end of the first steering wire to the first steering wire and fixating the proximal end of the second steering wire to the second steering wire.

15. A system comprising an endoscope according to any one of claims 1-13, a monitor and a control unit.
